# EUROPEAN PATENT SPECIFICATION

(11) **EP 1 659 921 B1**
(45) Date of publication and mention of the grant of the patent: **11.05.2016**
(21) Application number: 04780129.5
(22) Date of filing: 04.08.2004
(51) Int. Cl.: A61B 1/00

(54) **SHEATH WITH CHANNEL FOR ENDOSCOPE**
SCHLEUSE MIT KANAL FÜR ENDOSKOP
GAINE A CANAL POUR ENDOSCOPE

(30) Priority: 04.08.2003 US 491971 P
(43) Date of publication of application: 31.05.2006
(60) Divisional application: 14198653.9
(73) Proprietor: Vision-Sciences, Inc., Natick, MA 01760 (US)
(72) Inventor: MARTONE, Stephen, Westford, MA 01886 (US); HADANI, Ron, Teaneck, NJ 07660 (US)
(74) Representative: Nederlandsch Octrooibureau
(86) International application number: PCT/US2004/025238
(87) International publication number: WO 2005/016181

(56) References cited:
- WO-A1-95/18562
- US-A- 4 406 656
- US-A- 5 025 778
- US-A- 5 025 778
- US-A- 5 217 001
- US-A- 5 377 688
- US-A- 5 489 256
- US-A- 5 503 616
- US-A- 5 588 432
- US-A- 5 810 776
- US-A1- 2001 000 040
- US-B1- 6 358 200
- US-B1- 6 461 294
- US-B1- 6 808 520
- US-B2- 6 761 685

## Description

### FIELD OF THE INVENTION

The present invention relates to sheaths for medical apparatus.

### BACKGROUND OF THE INVENTION

Endoscopes are used to view internal tissue of humans, and for many other tasks. As sterilization of endoscopes is relatively difficult, disposable sheaths which cover an endoscope are used to isolate the endoscope from the patient tissue, so as to avoid time-consuming cleaning and disinfection processes. In some cases it is desired to have one or more channels run along the endoscope. These channels may be used, for example, to pass tools and fluids to the tip of the endoscope. As the sheath should completely isolate the endoscope from the human tissue, such channels are generally attached to the sheath, so that they are on an outer side of the endoscope. This, however, enlarges the cross-section of the sheath-covered endoscope being inserted into the patient. Such a larger diameter may make the insertion of the endoscope more difficult or may prevent the insertion altogether.

U.S. patent 5,025,778 to Silverstein et al., the disclosure of which is incorporated herein by reference, describes a sheath having an elastic double wall portion which is inflatable (stretchable) to form a channel after the endoscope is inserted into the patient. The 5,025,778 patent also describes an embodiment of an inflatable sheath entirely surrounding an endoscope, which is inflatable to form an annular channel. The inflating of the channel is problematic, however, especially when the endoscope is inserted to a tight tissue location. Furthermore, even if the inflating of the channel is possible, the forces involved in inflating the channel may distort the layout of the endoscope. Also, the tendency of the channel to return to its relaxed state adds significant frictional resistance to movement of devices through the channel.

WO 95/18562 to Jones discloses a channel system for use with a conventional endoscope. The system comprises a collapsible access channel that is connected to a first distal end of the endoscope and extends alongside and exterior to the cylindrical portion of the endoscope. The collapsible channel includes an access opening communicating between the two ends of the collapsible channel.

US 5,810,776 to Bacich et al. discloses a laparoscopic introducer and method of use comprise a laparoscopic channel and a guide channel for a secondary instrument. Upon initial insertion of the introducer, the guide channel closely conforms to the body of the introducer so as to only negligibly increase the cross-sectional profile thereof. The laparoscopic introducer is constructed from an inner tubing of stainless steal which compress the laparoscopic channel. Surrounding the inner tubing is a split nylon tube which couples the guide channel to the stainless steel tubing of the introducer.

US 5,217,001 to Nakao et al. discloses a device utilizable with an endoscope to promote sterility comprising a substantially thin-walled elongate tubular sheath made of a flexible material such as rubber, the sheath having a use configuration with an inner diameter larger than an outer diameter of an insertion member of the endoscope, whereby the sheath may be removably disposed about such insertion member to substantially surround same prior to insertion of the insertion member into a patient. The device further comprises a transparent cap member and an element or elements for attaching the cap member in a fluid tight seal to a distal end of the sheath so that the cap member covers a distal end of the insertion member of the endoscope. Biopsy channels, some of which are expandable from collapsed configurations to receive endoscopic surgical instruments during an operation, are provided on or in the sheath.

US 4,406,656 to Hattler et al. discloses a venous catheter having a central flexible lumen with a formed fluid passageway therein and a plurality of collapsible lumens mounted around the periphery of the central flexible lumen also having formed fluid passageways therein. The central flexible lumen being formed of material which is capable of retaining its shape with or without fluid flowing therethrough. Each collapsible lumen being formed from material which is normally collapsed in a small cross-sectional area and which is further capable of expanding when fluid is flowing therein to a cross-sectional area much greater than that when collapsed.

US 2001/0000040 A1 to Adams et al. discloses (paragraphs 0060-0062) an endoscope with a stiffening member disposed to the outside of the endoscope and adjacent a lumen provided along the endoscope. The stiffening member may include a sheath guide fixedly disposed to the outside of the endoscope. A guide pin may be brought in or out contact with the sheath guide to control a deflection of the lumen at the distal end of the endoscope.

### SUMMARY OF THE INVENTION

The present invention relates to an endoscopic sheath assembly, as defined in claim 1. It may include a foldable channel. The envelope material defining the channel is referred to herein as a tube. During insertion of the endoscope, the foldable channel is folded, such that its volume does not substantially add to the cross-section area of the endoscope and sheath. After insertion, the channel is unfolded so that fluid, tools and/or a working tube (referred to in the art also as a working channel) may be passed through the circumference of the additional sheath is greater than the circumference of the inner sheath by at least 0.5 mm or even 0.7 mm. In some embodiments of the invention, the additional sheath has an inner diameter greater by at least 10% or even 20% than the outer diameter of the inner sheath.

In some embodiments of the invention, the sheath assembly includes a plurality of foldable channels or one or more inflatable channels and one or more foldable channels.

In some embodiments of the invention, the additional sheath is open at its distal end so that the channel defined between the inner sheath and the additional sheath can be used to lead fluids into and/or out of the patient. Alternatively, the additional sheath is closed at its distal end, for example when there are holes along the length of the channel.

In the following description the term foldable channel is taken to refer to a channel whose volume can be reduced by folding the channel walls, such that the perimeter of the walls is mostly the same length in the folded state as in the open state. The terms inflatable channel and stretchable channel refer to channels that have a reducible volume due to a substantial reduction in the perimeter of the walls of the channel in the closed state.

An aspect of some embodiments of the present invention relates to a sheath assembly for an endoscope which includes at least two sheaths, one of which is located within the other, which are partially longitudinally attached to each other. The volume between the sheaths is used as a channel of the sheath assembly. Partially connecting the sheaths controls the path of tools or working tubes when inserted into the channel.

Optionally, the sheaths are attached to each other along at least one longitudinal line running along substantially the entire length of the sheath assembly. Alternatively, the sheaths are attached only along a portion of their longitudinal length.

In some aspects of the disclosure the sheaths are attached along a plurality of separate longitudinal lines. Alternatively or additionally, the sheaths are connected along at least a third or even half the circumference of the sheaths.

Some aspects of the disclosure relate to a sheath assembly including a channel having a variable transverse extent with a nozzle at its distal end. The channel optionally includes a foldable channel or an inflatable channel. The nozzle is optionally used to direct a fluid passing through the channel in a specific direction, for example to wash a viewing window of the endoscope.

An aspect of some embodiments of the present invention relates to an endoscopic sheath adapted to allow insertion of a working tube into a channel defined by the sheath. The working tube defines an external protrusion and the sheath includes a respective groove for receiving the protrusion, running along its length. In an exemplary embodiment of the invention, the external protrusion comprises a dovetail and the groove comprises a partially closed notch which prevents annular escape of the protrusion from the groove. Alternatively, annular escape is prevented by the tight fitting of the working tube into the relatively flexible channel. The groove may run over the entire length of the sheath or may run over a portion (e.g., a proximal portion) of the sheath.

Some aspects of the present disclosure relate to a method of inserting a working tube into a channel defined by an endoscopic sheath with a variable transverse extent, using a guide wire running along the channel. In some embodiments of the invention, the guide wire is preinserted in the channel, for example at the time of manufacture of the sheath, so that there is no need to separately insert the guide wire into the patient. Alternatively, the guide wire is inserted into the channel after the sheath is inserted into the patient, so that the guide wire does not interfere in inserting the sheath into the patient.

In some embodiments of the invention, the sheath is inserted into a patient with a guide wire threaded through a distal end of the sheath. The ends of the guide wire extend through the proximal end of the sheath. In inserting the working tube into the flexible channel, the working tube is connected to a first end of the wire. The second end of the wire is pulled to bring the working tube to move into the channel. Alternatively, the guide wire is anchored to a distal end of the sheath.

An aspect of some embodiments of the invention relates to a sheath assembly for covering an elongate probe, that defines two or more annular channels, around the elongate probe. Optionally, the sheath assembly includes three or more sheaths surrounding each other. Alternatively or additionally, a channel is defined between an inner sheath of the assembly and the elongate probe.

In some embodiments of the invention, one or more of the sheaths is elastic, allowing its expansion in creating a channel for use in a medical procedure. Alternatively or additionally, one or more of the outer sheaths is sufficiently larger than a sheath that it encompasses, so as to define a channel of sufficient size. An aspect of some embodiments of the invention relates to a system for sensory threshold testing, which includes an annular channel that leads test air puffs to a tested site, such as the upper aero digestive tract. Using an annular channel allows mounting a tube defining the channel on an endoscope or any other invasive tool, so as to allow proper positioning of the distal end of the channel, while keeping a relatively small cross section for the system.

Optionally, the test air puffs are generated under the control of a controller that sets a time duration and a pressure of the generated puffs. In some embodiments of the invention, the controller is provided with an indication of a type or attribute of a channel leading the puffs to their destination and accordingly sets the time duration and/or the pressure of the puffs.

An aspect of some embodiments of the invention relates to a sheath assembly for an endoscope or other elongate invasive medical tool, on which an electrode is mounted. The electrode is mounted on a radially expandable portion of the sheath assembly. During insertion of the endoscope, the portion carrying the electrode is not expanded in order to minimize the stresses of internal contact with the patient, on the electrode. For use, the portion carrying the electrode is expanded radially to bring the electrode into contact (or enhance the contact) with the body tissue.

Optionally, the portion carrying the electrode is unfolded in order to expand radially. Alternatively or additionally, the portion carrying the electrode is inflated.

There is therefore provided in accordance some aspects of the disclosure, a sheath assembly for an invasive probe, comprising an internal sheath for covering a probe and at least one channel tube external to the internal sheath, the channel tube being foldable into a closed state in which the tube does not define a channel, or openable into an open state in which the tube defines a channel that extends along a portion of the sheath assembly.

Optionally, the channel tube when in a closed state does not unfold from the closed state absent an external force. Optionally, the channel tube is folded in an unorganized manner in the closed state. Alternatively or additionally, the channel tube is folded in an organized manner in the closed state. Further alternatively or additionally, the channel tube is pleated in the closed state.

Optionally, the channel tube is folded over the internal sheath, in the closed state. Optionally, the channel tube is self-collapsible, such that it does not remain in the open state, without a force not due to the channel tube that holds it in the open state. Optionally, the channel tube does not self-collapse out of the open state, unless an external force is applied to the channel tube. Optionally, the channel tube is deformed in a manner which prevents self-collapsing out of the open state. Optionally, the channel tube is heat-set in the closed state so as to remain in the closed state until being moved to the open state.

Optionally, the channel tube is held in the closed state by an adhesive so as to remain in the closed state until being moved to the open state. Optionally, the channel tube surrounds the internal sheath. Alternatively, the tube does not surround the internal sheath. Optionally, the channel tube is directly attached to the internal sheath. Optionally, over most of the length of the sheath assembly the external sheath is not attached to the internal sheath.

Optionally, over most of the length of the sheath assembly the external sheath is attached to the internal sheath along at least one longitudinal line. Optionally, the channel tube and the internal sheath are connected, separately, to a proximal connector. Optionally, the channel tube is formed with an internal notch adapted to receive a dovetail of a working tube.

Optionally, the channel tube does not have an aperture at its distal end. Optionally, the channel tube has an aperture leading out of the sheath assembly, along its length. Optionally, the channel tube is formed with a foldable lobe of a limited axial extent relative to the channel tube, mounted on the channel tube and open to the channel defined by the channel tube. Optionally, the sheath includes an electrode mounted on an external surface of the channel tube. Optionally, the at least one channel tube extends over at least 50% of the internal sheath.

There is further provided in accordance with some aspects of the disclosure, an invasive tool, comprising an elongate probe and at least one flexible channel tube, for coupling to the elongate probe, the channel tube being foldable into a closed state in which the tube does not define a channel, or openable into an open state in which the tube defines a channel that extends along a portion of the sheath assembly, the channel tube is held in the closed state, absent a force that moves the channel tube to the open state. Optionally, the channel tube is heat set in the closed state.

Optionally, the channel tube is fixed in the closed state by an adhesive. Optionally, the invasive tool includes an internal sheath slid over the elongate probe and wherein the at least one channel tube is attached to an external surface of the internal sheath. Optionally, the invasive tool includes an electrode mounted on an external surface of the channel tube. Optionally, the channel tube is non-elastic.

There is further provided in accordance with some aspects of the disclosure, a channel add-on for an invasive probe, comprising at least one channel tube, for coupling to an invasive probe, which is foldable into a closed state in which the tube does not define a channel, or openable into an open state in which the tube defines a channel and means for opening the tube into the open state while the tube is within the patient.

Optionally, the means for opening the tube comprise means for dissolving an adhesive and/or means for injecting a fluid into the tube.

There is further provided in accordance with some aspects of the disclosure, a method of providing an endoscopic channel, comprising inserting into a patient, a probe with a sheath assembly including a channel tube being foldable into a closed state in which the tube does not define a channel, or openable into an open state in which the tube defines a channel that extends along a portion of the sheath assembly and opening the tube into the open state while the tube is within the patient.

Optionally, moving the tube into the open state comprises inserting a working tube or a tool into the tube. Optionally, moving the tube into the open state comprises dissolving an adhesive holding the tube folded. Optionally, moving the tube into the open state comprises injecting a fluid into the tube.

Optionally, inserting the probe comprises inserting while the channel tube is held in the closed state. Optionally, inserting the probe comprises inserting while the channel tube is not held in any specific state. Optionally, the channel tube surrounds the probe. Optionally, inserting the probe comprises inserting a probe surrounded by an internal sheath. Optionally, the channel tube is self-collapsible from the open state. Alternatively, the channel tube is non-self-collapsible from the open state.

There is further provided in accordance with an exemplary embodiment of the invention, a sheath assembly for a probe, comprising an internal sheath configured to isolate a probe from body fluids and an external sheath surrounding the internal sheath, the internal and external sheaths being directly connected to each other.

Optionally, the internal and external sheaths are connected to each other over at least one axial line extending over a segment of the length of the sheaths. Optionally, the internal and external sheaths are connected over at least two longitudinal lines, so as to define a plurality of separate channels between the sheaths. Optionally, the internal and external sheaths are connected non-symmetrically radially. Optionally, the internal and external sheaths are connected radially symmetrically. Optionally, the internal and external sheaths are connected substantially only at a plurality of circumferential points at a distal end of the external sheath. Optionally, the internal and external sheaths coextend at their distal ends, such that their distal ends extend to a same point.

Optionally, the internal sheath extends beyond the distal end of the external sheath.

There is further provided in accordance with some aspects of the disclosure, a sheath assembly for a probe, comprising an intermediate sheath configured to define a first channel between the probe and the intermediate sheath; and an external sheath adapted to define a second channel between the intermediate sheath and the external sheath.

Optionally, the sheath assembly includes a proximal port connected to the first channel. Optionally, the sheath assembly includes an internal sheath configured to isolate the probe from body fluids. Optionally, at least one of the intermediate sheath and the external sheath is stretchable so as to define the respective channel. Optionally, at least one of the intermediate sheath and the external sheath includes loose material that can be unfolded to define the respective channel.

There is further provided in accordance with an exemplary embodiment of the invention, a sheath assembly for a probe, comprising an internal sheath for covering an elongate probe, a channel tube with a variable transverse extent, external to the internal sheath and a nozzle connected to the distal end of the channel tube.

Optionally, the channel tube comprises a foldable channel. Alternatively, the channel comprises a stretchable channel. Optionally, the nozzle is directed in a direction substantially different from the main axis of the distal end of the channel. Optionally, the sheath assembly includes a window at the distal end of the internal sheath and wherein the nozzle is directed in a direction suitable for flushing the window.

There is further provided in accordance with an exemplary embodiment of the invention, a sheath assembly for a probe, comprising an internal sheath for covering an elongate probe, a channel tube with a variable transverse extent, external to the internal sheath, the channel tube not having an aperture at its distal end.

Optionally, the sheath assembly includes one or more holes along an axial length of the channel tube.

There is further provided in accordance with an exemplary embodiment of the invention, a sheath assembly, comprising an endoscopic tube defining a channel with a variable transverse extent, including a longitudinal notch formed in the tube; and a working tube comprising a protrusion adapted to fit into the notch.

Optionally, the protrusion has a dovetail shape. Optionally, the endoscopic tube comprises a foldable tube. Alternatively, the endoscopic tube comprises an inflatable tube.

There is further provided in accordance with an exemplary embodiment of the invention, a method of inserting a working tube into an endoscopic channel, comprising providing a guide wire within the channel, within a patient and inserting the working tube into the channel along the guide wire.

Optionally, providing the guide wire comprises providing the guide wire in the channel before the channel is inserted into the patient. Alternatively, providing the guide wire comprises providing the guide wire in the channel after the channel is inserted into the patient. Optionally, providing the guide wire comprises providing the guide wire such that both ends of the guide wire extend out of a proximal end of the channel. Optionally, providing the guide wire comprises providing a guide wire that is anchored to a distal end of the channel. Alternatively or additionally, providing the guide wire comprises providing a guide wire that is threaded through a distal end of the channel.

### BRIEF DESCRIPTION OF THE DRAWINGS

Exemplary non-limiting aspects of the disclosure will be described with reference to the following description in conjunction with the figures. Identical structures, elements or parts which appear in more than one figure are preferably labeled with a same or similar number in all the figures in which they appear, and in which:
Fig. 1A is a schematic side view of a sheath assembly;
Fig. 1B is a cross-sectional view of the sheath assembly of Fig. 1A;
Figs. 2A and 2B are schematic cross-sectional views of a sheath assembly, in closed and open positions;
Fig. 3 is a flowchart of acts performed in using a sheath assembly;
Figs. 4A and 4B are schematic cross-sectional views of a sheath assembly in closed and open positions;
Fig. 5 is a schematic cross-sectional view of a sheath assembly in a closed position;
Figs. 6A and 6B are side and isometric views of a sheath assembly;
Fig. 7 is an end view of an endoscope sheath assembly and compatible working tube with a dovetail, in accordance with an exemplary embodiment of the invention;
Fig. 8 is a side view of a sheath assembly;
Fig. 9 is a schematic illustration of insertion of a working tube into a channel;
Fig. 10 is a schematic cross sectional view of a sheath assembly;
Figs. 11A and 11B are side sectional views of a sheath assembly in a closed state and an open state, respectively; and
Fig. 12 is a schematic illustration of a system 900 for sensory discrimination threshold testing.

### DETAILED DESCRIPTION OF EXEMPLARY EMBODIMENTS

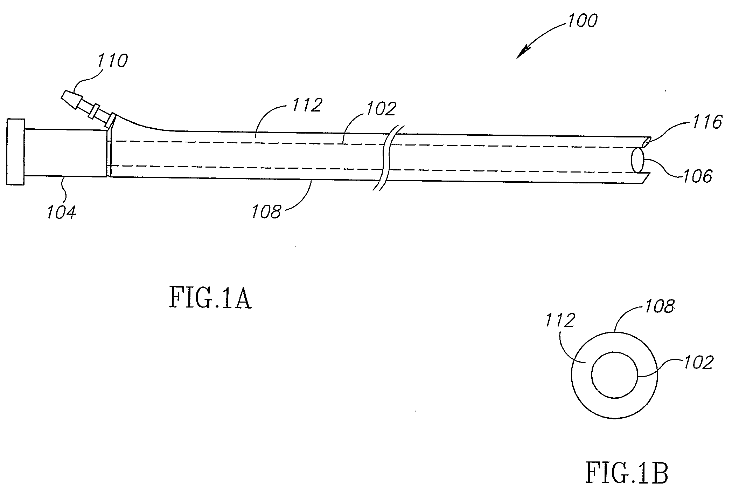

Figs. 1A and 1B are a schematic side view and a cross sectional view of a sheath assembly 100, in accordance with an aspect of the disclosure. Assembly 100 optionally includes an internal sheath 102 adapted to receive an endoscope and isolate the endoscope from the environment. In some aspects of the disclosure, a rigid pipe section 104 is located at a proximal end of internal sheath 102, to aid insertion of the endoscope into the sheath. A sealed window 106 at the distal end of internal sheath 102, optionally isolates the endoscope from the environment while allowing a camera or fiberoptic image bundle of the endoscope to provide images of the tissue external to sheath assembly 100.

An external sheath 108, having a larger circumference than internal sheath 102, optionally surrounds internal sheath 102. During insertion of an endoscope with sheath assembly 100 into a patient, external sheath 108 is optionally closely folded around internal sheath 102, such that the cross-sectional area of an endoscope with sheath assembly 100 is not substantially enlarged by the inclusion of external sheath 108. After sheath assembly 100 is inserted into the patient, external sheath 108 is unfolded, to form a channel 112 in the area between internal sheath 102 and external sheath 108, such that at least a portion of external sheath 108 serves as a channel tube defining channel 112.

In an aspect of the disclosure, internal sheath 102 has an outer diameter of between about 3.5-3.7 mm and the inner diameter of external sheath 108 is larger than the outer diameter of internal sheath 102 by about 0.25 mm. In other aspects of the disclosure, smaller and/or larger diameters of the inner sheath, are used. Optionally, the inner diameter of internal sheath 102 is selected to snugly fit over the endoscope. Alternatively, internal sheath 102 has a larger diameter than the endoscope, such that the volume between internal sheath 102 and the endoscope can be used for a working channel and/or to allow relatively easy sliding of internal sheath 102 over the endoscope.

In some aspects of the disclosure, the inner diameter of external sheath 108 is larger than the outer diameter of internal sheath 102 by more than 0.25 mm, for example by between about 0.5-1mm or even more, depending on the usage of the channel. In an aspect of the disclosure, the diameter of the external sheath 108 is greater than the diameter of internal sheath 102 by between about 2-3 mm, for example 2.5 mm, so that a sufficiently large working tube, can be located in or passed through channel 112. For example, channel 112 may be used to carry a suction tube, such as described in US provisional patent application 60/503,780, titled "Braided Minimally Invasive Channel" and filed September 18, 2003,

Sheaths 102 and 108 have a thickness of between about 0.03 to 0.4 mm, for example between about 0.05 to 0.12 mm. Alternatively, thicker or thinner sheath materials may be used. In some aspects of the disclosure, both the internal and external sheaths have substantially the same thickness and are formed from the same material. Alternatively, external sheath 108 is thicker or otherwise stronger (e.g., formed of a different material) than internal sheath 102, so as to endure the stresses of internal contact with the patient's body. Further alternatively, the internal sheath is stronger than the external sheath, for example when it is used for isolating the endoscope.

Channel 112 is optionally used to provide fluids to the distal end of sheath assembly 100. Alternatively or additionally, channel 112 is used for introducing accessory devices. Further alternatively or additionally, a working tube is introduced to the patient through channel 112. Optionally, the working tube is relatively rigid, so that the channel does not collapse on the working tube.

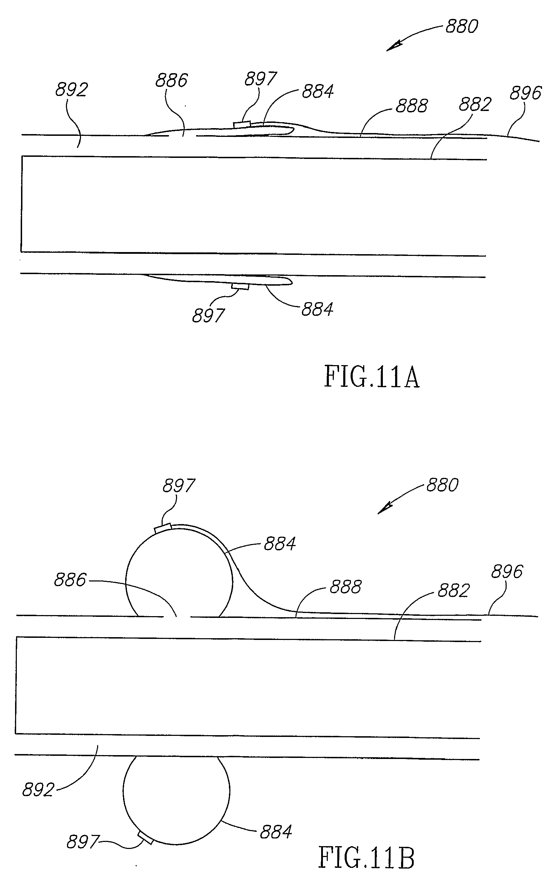

The size difference between the circumferences of internal sheath 102 and external sheath 108 is optionally chosen according to a desired size of channel 112. In an aspect of the disclosure, the size of the channel 112 is chosen according to a desired impedance for fluids passing through the channel. In some aspects of the disclosure, the size of channel 112 is chosen as a compromise between maximizing the fluid impedance and minimizing the cross-sectional area of sheath assembly 100. Alternatively or additionally, the size of channel 112 is set to achieve a predetermined fluid impedance, for example as described below with reference to Fig. 11.

Sheath 108 optionally comprises an elastic material, such as polyurethane or polyvinylchloride with a sufficiently large amount of added plasticizer, that can bend longitudinally around corners while the sheathed endoscope is inserted into the patient. Alternatively, the material of external sheath 108 is relatively non-elastic, e.g., Polyethyleneterephtalate (PET), polyvinylchloride with a relatively small amount of added plasticizer, or a very thin (e.g., between about 0.05-0.1 mm) layer of Teflon or Polyethylene, as relatively non-elastic materials are generally more suitable for folding and for passing tubes and/or tools through them, due to their relative stiffness. In some aspects of the disclosure, the elasticity of the material is chosen as a compromise between the desire for smooth bending and the easier folding and passing of tools.

In some aspects of the disclosure, sheath 108 is formed in a self-collapsible manner, such that when not held open, channel 112 closes. Alternatively, sheath 108 is formed in a non-self-collapsible manner, such that once opened channel 112 does not close unless a force to induce the collapse is applied to the channel. For example, the material of sheath 108 may be deformed in a predetermined shape, as is known in the art of stents, such that it does not collapse after being unfolded. Optionally, sheath 108 is deformed over its entire length. Alternatively, sheath 108 is deformed in one or more locations along its length, which locations are sufficient to prevent collapse of channel 112. Further alternatively, stent-like structures are embedded within sheath 108 along its length in order to prevent collapse after it is unfolded.

Internal sheath 102 optionally comprises the same material as sheath 108 possibly allowing a simpler production procedure. Alternatively, internal sheath 102 and external sheath 108 comprise different materials. For example, internal sheath 102 may comprise a thinner or weaker material as it is less affected by the forces involved in inserting the sheath assembly to a patient. Alternatively, internal sheath 102 is relatively rigid, for example reinforced by relatively rigid rings, in order to prevent inner sheath 102 being affected when channel 112 is being used.

In some aspects of the disclosure, the entire annular cross-section between internal sheath 102 and external sheath 108 forms channel 112 and is open, for example, for flow of fluids. Using an annular channel is relatively immune against blockage due to bending of sheath 108, as a bend in one direction still allows passage of fluids on the opposite side of the annular channel. Alternatively, internal sheath 102 is fastened to external sheath 108 along one or more longitudinal lines or portions. The fastening of the internal sheath 102 to external sheath 108 optionally limits the size of channel 112. Alternatively or additionally, the fastening of sheaths 102 and 108 to each other does not necessarily limit the size of channel 112, but simplifies the combined insertion of the sheaths and/or prevents distortion of the sheath assembly during insertion. The fastening of the internal sheath 102 to external sheath 108 optionally also prevents a working tube or tool passed through channel 112 from inadvertently wrapping around the inner sheath during insertion to the channel.

Alternatively to including a single channel 112, in some aspects of the disclosure, channel 112 is divided along its entire length into a plurality of sub-chaimels. Further alternatively or additionally, channel 112 is divided into a plurality of sub-channels over only a portion of the length of sheath assembly 100. For example, when the separate sub-channels are used for leading separate working tubes, the sub-channels are optionally defined at the proximal end of the channel, while at the distal end channel 112 is not divided into sub-channels.

In an aspect of the disclosure, the distal end of channel 112 is formed into a nozzle 116, which directs fluid passing through the channel in a specific direction as it exits the channel. For example, the nozzle may be directed toward window 106, so the fluid can keep window 106 clean. Alternatively, the nozzle may be directed in a specific direction which is best suited for directing fluids at tissue of the patient. Alternatively to forming the end of internal sheath 102 and/or external sheath 108 into a nozzle, the end of channel 112 is connected to an external nozzle (not shown). The external nozzle is optionally produced separately from the sheaths and is connected to the sheaths after they are produced. Alternatively or additionally, the external nozzle is not produced from the same material as the sheaths.

In some aspects of the disclosure, nozzle 116 comprises a rigid material which, during insertion into the patient, is bent in front of window 106, in order not to add to the diameter of sheath assembly 100 during the insertion. Nozzle 116 is optionally kept in its bent position using an adhesive or a sticky material. In some embodiments of the invention, the adhesive is detached by injecting a fluid through channel 112. Alternatively, nozzle 116 comprises a flexible material which is folded together with external sheath 108. In some aspects of the disclosure, nozzle 116 is constructed as a single piece with external sheath 108. For example, nozzle 116 may include a continuous portion of external sheath 108 slightly extending beyond window 106.

Alternatively, the distal end of channel 112 does not include a nozzle. In some embodiments of the invention, the entire cross section of channel 112 is open at the distal end. Alternatively, a portion of external sheath 108 is sealed to internal sheath 102 at the distal end of channel 112, so that fluids can exit and/or enter the channel 112 from specific portions only. Further alternatively or additionally, the distal end of channel 112 is entirely sealed, for example when channel 112 has holes along its length for dispersing liquids. In some aspects of the disclosure, the distal end of channel 112 is sealed during insertion and is later punctured for use.

In some aspects of the disclosure, channel 112 includes one or more holes along the length of the channel, allowing the provision of fluids from points along the length of the channel. Such holes are used, for example, for dispensing a lubricant or drug.

A proximal tube 110 optionally serves as an interface to channel 112. Optionally, when channel 112 is divided into a plurality of sub-channels, each sub-channel has a separate proximal tube. The proximal ends of external sheath 108 are optionally attached to proximal tube 110 and/or are sealed to proximal ends of internal sheath 102 or to rigid pipe 104, such that the only entrance point to channel 112 is through proximal tube 110.

In some aspects of the disclosure, internal sheath 102 and external sheath 108 are not attached to each other directly. Optionally, sheaths 102 and 108 are attached to each other indirectly through proximal tube 110 and/or through any other proximal connector or port. Alternatively or additionally, for example as described below, sheaths 102 and 108 are connected at their distal end. Further alternatively or additionally, sheaths 102 and 108 are connected along their length, for example in order to define sub-channels, as suggested above.

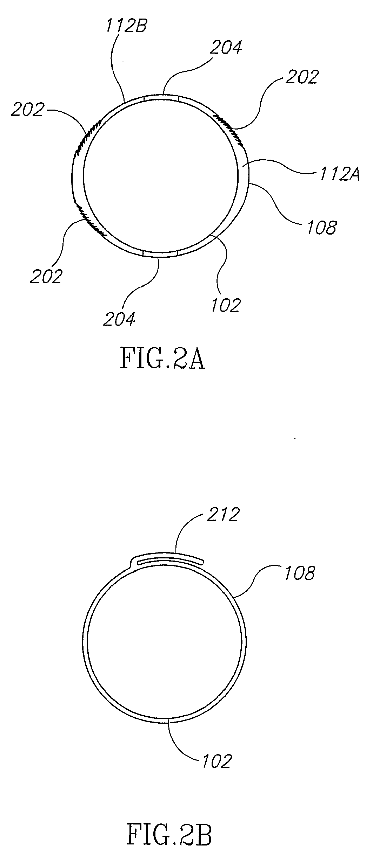

Fig. 2A is a schematic cross-sectional view of sheath assembly 100 in a closed position, in accordance with an aspect of the disclosure. In Fig. 2A, the material of external sheath 108 beyond that required to tightly surround internal sheath 102 is folded by pleating in one or more locations 202 around the cross-section of external sheath 108. In Fig. 2A, external sheath 108 is spaced from internal sheath 102, for clarity. In some embodiments of the invention, the folds included in pleating locations 202 partially overlap, such that the folds are generally parallel to the surface of internal sheath 102. Alternatively or additionally, in one or more pleating locations, the folds entirely overlap, optionally being oriented partially or entirely perpendicular to the surface of internal sheath 102.

In the aspect of the disclosure of Fig. 2A, external sheath 108 is connected to internal sheath 102 along two longitudinal strips 204, forming two channels 112A and 112B. As shown, the connection of external sheath 108 to internal sheath 102 is symmetric, so as to prevent deformation of the sheath assembly while insertion of the sheathed endoscope into the patient. Alternatively, the connection of the internal and external sheaths is non-symmetric, forming channel sizes most suitable for use. Optionally, the number of pleats in each location 202 depends on the intended size of the channel 112B, in an open state. Alternatively, the number of pleating locations 202 in each channel 112 depends on the intended size of the channel. As shown, the left channel 112B is intended to be larger after opening than right channel 112A and therefore includes two pleated locations 202.

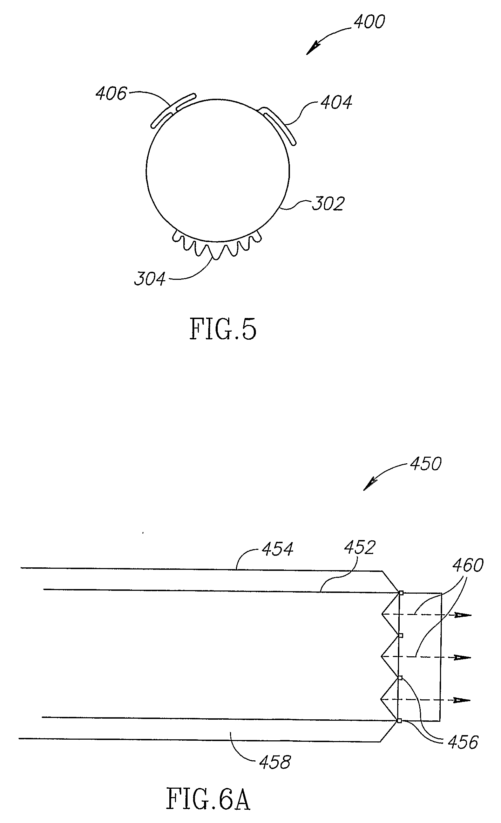

Fig. 2B is a schematic cross-sectional view of sheath assembly 100 in a closed position. The additional material of external sheath 108, required for channel 112, is included in a portion 212 folded over the circumference of external sheath 108. Alternatively, to the additional material of external sheath 108 being folded only in one direction, as shown in Fig. 2B, the additional material may be folded in both directions, optionally evenly (as shown for example in Fig. 5). The use of a single bend, as in Fig. 2B, rather than a pleating, as in Fig. 2A, reduces the amount of bending, which may weaken external sheath 108, applied to the external sheath. Pleating, on the other hand, is more easily unfolded than a relatively large bended portion. In some aspects of the disclosure, the number of folds in the pleating is chosen as a compromise between the strength of the material of external sheath 108 and the allowance of relatively easy passive unfolding.

In some aspects of the disclosure, the pleating and/or other folding are kept in place by an adhesive strong enough to withhold the forces involved in inserting sheath assembly 100 into the patient, but not too strong to prevent intentional opening of channel 112.

Alternatively to folding the additional sheath portion of external sheath 108 while assembly 100 is inserted into the patient, the sheath assembly may be inserted into the patient on the endoscope, while the additional portion of external sheath 108 is allowed to move freely. During insertion, external sheath 108 generally assumes the shape of the body cavity in which it passes. In some body locations, sheath 108 may be pushed at the endoscope from some radial directions, while allowed to lay freely in other radial directions. In some cases, external sheath assumes an oval cross-sectional shape, when some radial portions of external sheath 108 touch internal sheath 102, while in other radial portions external sheath 108 is distanced from the internal sheath. The portions of external sheath 108 that are distanced from internal sheath 102 may optionally change according to the body cavities through which the sheath assembly is passed. In some aspects of the disclosure, during the insertion of sheath assembly 100 to the patient, the external sheath 108 folds due to narrow body cavities through which the assembly passes.

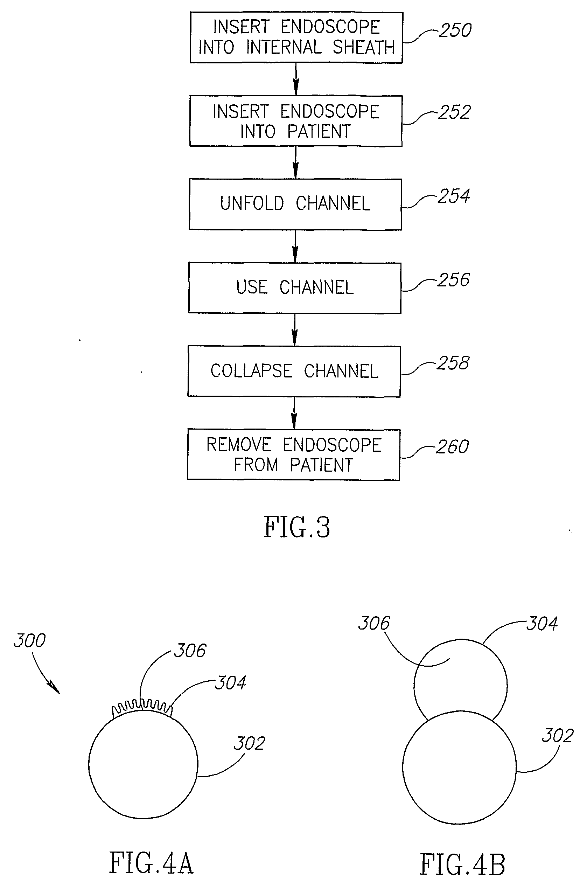

Fig. 3 is a flowchart of acts performed in using sheath assembly 100. An endoscope is optionally inserted (250) into internal sheath 102. The endoscope together with sheath assembly 100 is inserted (252) into the patient and guided to a desired position. Channel 112 is then unfolded (254) after the sheathed endoscope is in place. Channel 112 is then used (256) in performing a medical procedure. In some aspects of the disclosure, after the procedure, channel 112 is collapsed (258) and the sheathed endoscope is removed (260) from the patient.

Referring in more detail to unfolding (254) channel 112, in some embodiments of the invention, the unfolding is actuated by injecting a fluid into the channel at a suitable pressure.

In some aspects of the disclosure, the folds and/or pleating of the sub-channel are heat set at the time of production, so that they remain in their folded position until the folds are opened. Alternatively or additionally, suction is applied to channel 112 while the sheathed endoscope is inserted into the patient, so as to keep the channel in its closed state, while the sheathed endoscope is inserted.

Further alternatively or additionally, the folds and/or pleating of the sub-channel are held by an adhesive within channel 112 and the injected fluid is a fluid that weakens or dissolves the adhesive. For example, a water soluble adhesive may be used. Alternatively or additionally, the adhesive is sensitive to temperature and is counteracted by the temperature of the injected fluid. This alternative and others may be used both with adhesives within channel 112 and with adhesives on the outside of channel 112.

In some aspects of the disclosure, the folds and/or pleating are held in place by an adhesive which wears away a predetermined time after sheath assembly 100 is taken out of its packaging. For example, the adhesive may be soluble in air. The physician is required to insert (252) the sheathed endoscope into the patient within the predetermined time and then waits the remaining time until the channel opens up on its own or is easily opened due to dissolution of the adhesive. Alternatively, the adhesive is sensitive to the conditions within the patient and dissolves within a predetermined time after the insertion of sheath assembly 100 into the patient. The adhesive may dissolve, for example, due to the body temperature of the patient and/or due to body fluids.

Alternatively or additionally, the unfolding is actuated by passing a stylet (optionally having a floppy or round tip) or a working tube through channel 112, so as to mechanically open the channel. In some aspects of the disclosure, the extent to which channel 112 is opened is controlled by the physician according to the size required for the medical procedure. The opening extent is optionally determined by the size and/or shape of the stylet or working tube used to unfold the channel. The working tube or stylet may have substantially any suitable cross-sectional shape, including circular, oval, triangular and rectangular. In some aspects of the disclosure, the unfolding is performed in a plurality of steps. Optionally, a first narrow stylet is inserted to channel 112 and afterwards a wider working tube is inserted into the channel.

Further alternatively or additionally, any other unfolding method, such as any of the methods known in the art for use with angioplasty balloons, is used.

In some aspects of the disclosure, external sheath 108 is folded in a manner which allows for different extents of unfolding forming channels of different cross-section areas. For example, external sheath 108 may have different areas of pleating with different closing strengths, and the extent of unfolding is determined by the force exerted by the physician.

Optionally, the folds are planned such that after the channel is unfolded channel 112 does not collapse under regular conditions within the patient. Alternatively, the channel collapses when it is not held open by a fluid or a working tube within the channel. Optionally, when it is desired to remove the sheathed endoscope from the patient, the channel is allowed to collapse, for example by removing a working tube previously inserted into the channel.

Although the unfolding (254) of the channel was mentioned as a separate act from the use (256) of the channel, in some aspects of the disclosure, the unfolding is performed passively as part of the use of the channel.

Referring in more detail to collapsing (258) channel 112, in some aspects of the disclosure, in which channel 112 is non-self-collapsible, suction is applied to channel 112 in order to collapse the channel. Optionally, the suction is continuously applied throughout the removal of the sheathed endoscope from the patient. Alternatively, the suction is applied before the sheathed endoscope is removed and thereafter the channel remains sufficiently collapsed in order to allow its easy removal from the patient, without applying suction. Further alternatively or additionally, a cord running through channel 112 is used to pull the channel into a collapsed position. Alternatively or additionally, channel 112 collapses automatically when a working tube is removed from the channel. Further alternatively or additionally, the sheathed endoscope is removed without collapsing the channel as it is easier to remove the sheathed endoscope than to insert the sheathed endoscope.

Fig. 4A is a schematic cross-sectional view of a sheath assembly 300 in a closed position. In Fig. 4A, a main sheath 302 is devised to receive an endoscope. An additional folded sheath portion 304 defining a channel 306 is mounted on a side of main sheath 302. In some embodiments of the invention, the width along which main sheath 302 and sheath portion 304 are connected is relatively wide (e.g., close to the diameter of channel 306). Alternatively, the width along which main sheath 302 and sheath portion 304 are connected is relatively narrow, allowing both sheaths to define round channels. In some aspects of the disclosure, the channel is designed to have a thin cross-section, for example with an elliptical shape, such that a relatively large channel cross section area can be achieved, without extending too far away from the body of the endoscope.

Fig. 4B is a schematic cross-sectional view of sheath assembly 300 in an open position. After the sheathed endoscope is inserted into its place in the patient, folded sheath portion 304 is unfolded to bring channel 306 into a usable size.

Fig. 5 is a schematic cross-sectional view of a sheath assembly 400 in a closed position. Sheath assembly 400 includes a main sheath 302 and three side folded sheath portions 304, 404 and 406. Folded sheath portions 404 and 406 are folded over the side of main sheath 302, while folded portion 304 is pleated.

In some aspects of the disclosure, the structure of the sheath defining the channel and/or of the inserted working tube aids in the unfolding of the channel.

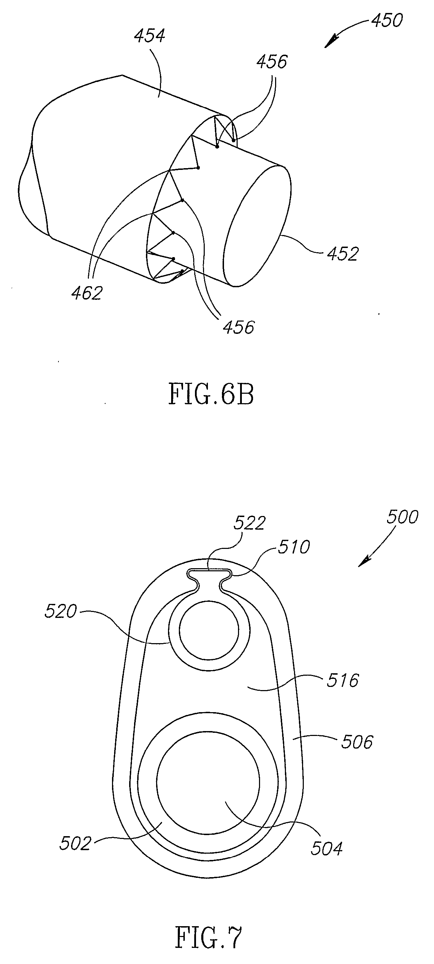

Figs. 6A and 6B are side and isometric views of a sheath assembly 450. Sheath assembly 450 comprises an internal sheath 452 for covering an endoscope, and an external sheath 454, which defines a circumferential channel. Optionally, external sheath 454 does not extend distally to the end of internal sheath 452, but rather extends up to several millimeters before the distal end of the internal sheath. This option is advantageous, for example, when channel 112 is used to infuse a lubricating fluid. Alternatively, the distal end of external sheath 454 extends up to the distal end of internal sheath 452. This alternative is used, for example, when channel 112 is used to introduce a medication to a specific anatomical area. Further alternatively, the distal end of external sheath 454 extends beyond the end of internal sheath 452. In this alternative, the additional distal end of external sheath 454 can protect surrounding tissue from a working tube or other tool inserted into channel 112. Thus, the distal tip of sheath assembly 450 is atraumatic.

In some aspects of the disclosure, external sheath 454 is coupled to internal sheath 452 at the distal end of the external sheath. External sheath 454 and internal sheath 452 are optionally not coupled along their length, but rather only at the distal end. The coupling at the distal end is optionally performed at a plurality of bonding points 456 around the circumference of internal sheath 452. In some aspects of the disclosure the distal end of external sheath 454 has a saw tooth shape and the bonding points 456 are at the distal tips of the triangles of the saw tooth shape. Alternatively or additionally, the bonding is performed at points 462 between the triangles of the saw tooth shape. The coupling is optionally performed symmetrically, so as to prevent deformation of the sheath assembly while it is inserted into the patient.

In some aspects of the disclosure, in use, a working tube is inserted along a side of a channel 458 defined between internal sheath 452 and external sheath 454. The attachment at bonding points 456 is optionally sufficiently strong so as not to break during insertion of sheath assembly 450 into the patient, but allows breaking of bonding points 456 by an inserted working tube. Optionally, in inserting the working tube, the tube is brought to the distal end of internal sheath 452 by breaking one or more of bonding points 456.

Sheath assembly 450 is optionally used for urology applications, in which a fluid (e.g., water 460) is injected to the patient through channel 458, while an endoscope sheathed with assembly 450 is being inserted to the patient. The use of a plurality of bonding points 456 limits external sheath 454 from folding back on itself, while allowing injection of required (possibly relatively large) amounts of fluid. Sheath assembly 450 may also be used for other applications in which fluid injection is desired.

Alternatively or additionally, the portions of external sheath 454 between each two bonding points 456 have a folded state and an open state.

Fig. 7 is an end view of an endoscope sheath assembly 500, after opening, in accordance with an embodiment of the invention. Sheath assembly 500 comprises an internal sheath 502, optionally having a viewing window 504, and an external sheath 506. External sheath 506 defines a notch 510 adapted to receive a dovetail 522. After sheath assembly 500 is inserted into a patient, a working tube 520 including a dovetail 522 is inserted into a channel 516 defined between external sheath 506 and internal sheath 502. Working tube 520 is optionally inserted into channel 516 by fitting dovetail 522 into notch 510 at a proximal end of sheath assembly 500 and pushing the working tube toward the distal end of the sheath assembly. The use of dovetail 522 and corresponding notch 510 prevents working tube 520 from wrapping around internal sheath 502 while the working tube is inserted into channel 516.

Alternatively to notch 510 running up to the distal end of sheath assembly 500, notch 510 runs over a portion of the length of external sheath 506. The portion of external sheath 506 including notch 510 is optionally sufficiently long to properly guide working tube 520 into place even where external sheath 506 does not include notch 510. Alternatively, dovetail 522 does not extend over the entire length of working tube 520, but only extends over a portion, optionally a distal portion, of the working tube. Having dovetail 522 extend only over a portion of the working tube, reduces the drag due to friction when the working tube is inserted to the channel.

In some embodiments of the invention, notch 510 is defined by a portion of external sheath 506 which is reinforced so that it does not deform in normal conditions before and/or while dovetail 522 is inserted into the notch. Alternatively, for simplicity of production, the area of external sheath 506 defining notch 510 is not reinforced. In some embodiments of the invention, dovetail 522 is produced as an extension of working tube 520 from the same materials.

Alternatively to using a dovetail shaped protrusion, any other protrusion shape may be used including circular and triangular shapes which have a captive fit in a respective notch. Further alternatively, a simple indent which is held inside its respective notch by the tight fitting of the protrusion in the notch, is used.

Alternatively or additionally to using a protrusion and notch to lead the working tube into the channel, a guide wire may be used.

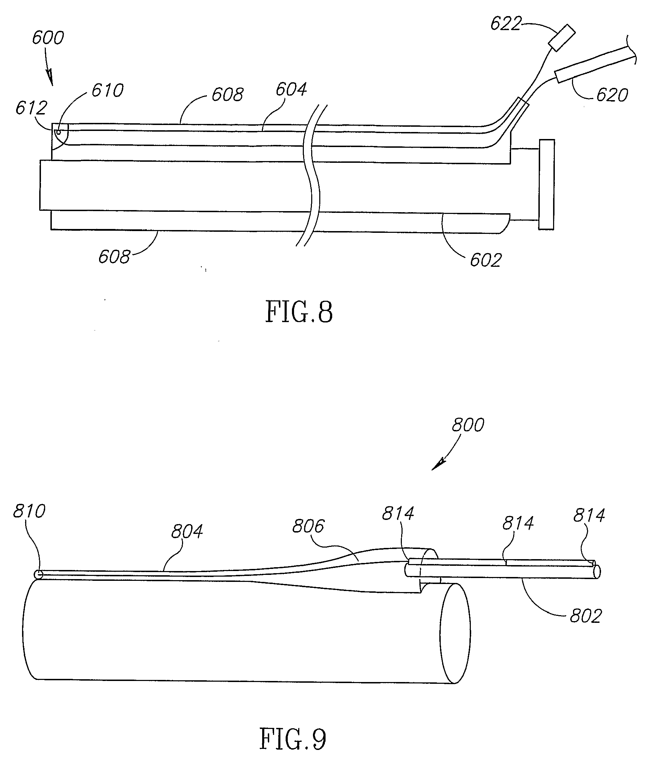

Fig. 8 is a side view of a sheath assembly 600. Sheath assembly 600 optionally includes an internal sheath 602 and an external sheath 608. A distal end of external sheath 608 optionally includes a reinforced portion 612 including an aperture 610. A guide wire 604 runs through aperture 610, with both its ends extending out of a proximal end of sheath assembly 600. One of the ends of guide wire 604 is optionally connected (e.g., at manufacture or by a physician at the time of use) to a working tube 620. Guide wire 604 is optionally connected to working tube 620 using any method known in the art, such as using an adhesive, thermal bonding, insert molding or mechanical or crimping attachment.

The other end of guide wire 610 is optionally connected to a handle 622 for pulling at the guide wire. After sheath assembly 600 is inserted into a patient, a physician optionally pulls at handle 622 so that guide wire 610 pulls working tube 620 into external sheath 608. The guide wire optionally remains within external sheath 608 throughout the entire procedure. Alternatively, the guide wire is removed from the channel after working tube 620 is inserted to the channel.

Fig. 9 is a schematic illustration of insertion of a working tube 802 into a channel 804 of a sheath assembly 800. Sheath assembly 800 optionally includes a guide wire 806 passing through channel 804. In some embodiments of the invention, guide wire 806 is anchored to a distal end of sheath assembly 800, for example at an anchor point 810. Working tube 802 optionally includes one or more loops 814 adapted to receive guide wire 806. Alternatively, working tube includes a small conduit through which guide wire 806 is passed. During insertion, working tube 802 is mounted on guide wire 806 and is pushed into channel 804. Using the guide wire prevents working tube 802 from tangling within channel 804. Optionally, after insertion, before the medical procedure, guide wire 806 is removed from the patient. In some aspects of the disclosure, the insertion of working tube 802 to the distal end of sheath assembly 800 releases the anchoring 810 of guide wire 806. Alternatively, the guide wire is not anchored so that it is easily removed from channel 804.

Alternatively to including the guide wire in the channel when sheath assembly 800 is inserted to the patient, the guide wire is inserted to the channel after the sheath assembly is within the patient. Further alternatively, a plurality of guide wires are inserted into channel 804, allowing organized insertion of a plurality of working tubes into a single channel.

Although Figs. 7, 8 and 9 were described with relation to sheath assemblies including two concentric sheaths, the methods of inserting a working tube into a channel of a sheath assembly may be used with other sheath assemblies, such as those described above with reference to Figs. 4A, 4B and 5 or those described in above mentioned U.S. patent 5,025,778. It is further noted that the methods of Figs. 7, 8 and 9 may be implemented with foldable sheaths, inflatable sheaths (e.g., sheaths that require stretching of their wall to expand) and/or any other self-collapsible or non-self-collapsible sheaths.

Furthermore, the methods of inserting a working tube may be used for insertion of other tools through an endoscopic channel. This method is especially useful when inserting a working tube into a channel having a variable transverse extent, due to its being foldable and/or elastic.

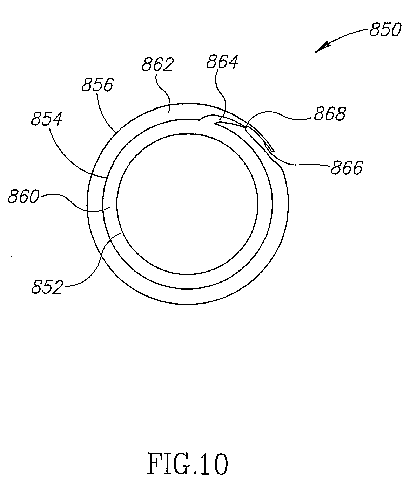

In some embodiments of the invention, a sheath assembly is formed of three or more concentric (or otherwise included within each other) sheaths which define between them a plurality of channels, as is now described with reference to Fig. 10.

Fig. 10 is a schematic cross sectional view of a sheath assembly 850. Sheath assembly 850 comprises an internal sheath 852, an intermediate sheath 854 and an external sheath 856. A channel 860 between internal sheath 852 and intermediate sheath 854 is optionally used for a first task, such as injecting fluids, while a channel 862 between intermediate sheath 854 and external sheath 856 is optionally used for a second task, such as suction.

In an aspect of the disclosure, an excess portion 864 of intermediate sheath 854 is folded over the intermediate sheath in a closed state. An excess portion 866 of external sheath 856 is folded inwards to a meeting point 868 with excess portion 864. Optionally, the excess portions are held together in a manner such that opening one of the channels 860 and 862 opens the other of the channels. For example, the excess portions 864 and 866 are optionally held by an adhesive which looses its bond when one of the channels is opened. Alternatively, any other method is used to open both channels concurrently. In some aspects of the disclosure, opening a first one of the channels (e.g., 860) opens the second channel (e.g., 862), while opening the second channel 862 does not open the first channel 860. Further alternatively, each of channels 860 and 862 is opened independently from the other channel.

In some aspects of the disclosure, the excess portions 864 and 866 are positioned at different locations around the circumference, so that there is no specific area with a double bulge. Alternatively, the excess portions are located on a same circumference area, in order to allow simpler combined release and/or in order to define bulging direction which is convenient for insertion into a non-symmetrical body cavity. In the above description, when channel 112 is open it extends in its open state along substantially the entire length of sheath assembly 100, at least within the patient. Thus, the unfolding of external sheath 108 enlarges the cross section of assembly 100 over substantially its entire length within the patient. In some cases, however, channel 112 may be required only up to an intermediate point along the length of an endoscope. In some such cases, external sheath 108 does not extend to the distal end of sheath assembly 100, but rather extends only up to the point at which channel 112 is required. Alternatively, external sheath 108 extends up to the distal end of sheath assembly 100. In some aspects of the disclosure, in accordance with this alternative, only the portion of channel 112 up to the required point is actively opened, while the remaining part is allowed to remain closed or open on its own.

In some aspects of the disclosure, channel 112 extends over at least 50%, 70% or even 90% of the length of the endoscope within the patient. In some cases, channel 112 extends over the entire length of the endoscope within the patient. Optionally, channel 112 extends continuously from the entrance of sheath assembly 100 into the patient to the distal point to which the channel extends. Alternatively or additionally, channel 112 has narrow axial sections and wider axial sections. In some aspects of the disclosure, in addition to channel 112, sheath assembly 100 includes an inflatable lobe or extension that extends over a limited axial portion of sheath assembly 100, as is now described with reference to Figs. 11A and 11B.

Figs. 11A and 11B are side sectional views of a sheath assembly 880 in a closed state and an open state, respectively. Sheath assembly 880 comprises an internal sheath 882, which is optionally adapted to receive an endoscope (not shown) and an external sheath 888 that surrounds internal sheath 882 and defines an annular channel 892 between the external and internal sheaths. A lobe 884 extends radially from external sheath 888. Lobe 884 optionally surrounds the entire circumference of external sheath 888. Alternatively, lobe 884 covers only a sector of the circumference of external sheath 888. Further alternatively, a plurality of adjacent or non-adjacent lobes extend radially at same or different axial positions along external sheath 888. A small aperture 886 optionally connects the interior of lobe 884 to channel 112.

Optionally, during insertion, lobe 884 is in a closed state, shown in Fig. 11A, in which lobe 884 is not inflated and the lobe is allowed to fold over external sheath 888. Optionally, in the closed state, lobe 884 is held against external sheath 888 by an adhesive. Alternatively, lobe 884 lies freely and folds back due to pressure of the body cavities in which sheath assembly 880 passes.

After sheath assembly 880 and the endoscope are properly positioned, lobe 884 is inflated, to an open state shown in Fig. 11B, for example by pumping a fluid into the lobe, through channel 892. In some aspects of the disclosure, aperture 886 is a simple aperture, which allows two way flow between the interior of lobe 884 and channel 892. In these aspects of the disclosure, the filling of lobe 884 with a fluid optionally includes filling channel 892, such that the channel is limited for use for other tasks. If necessary, an additional sheath may be used, as described above with reference to Fig. 10, for other tasks. Optionally, the additional sheath is located between the internal and external sheaths.

Alternatively to having a small aperture 886 to lobe 884, the aperture may be open over a substantial portion of the contact area between external sheath 888 and lobe 884. In some embodiments of the invention, lobe 884 is open to channel 892 over its entire radial contact with external sheath 888.

In some aspects of the disclosure, instead of a simple aperture, aperture 886 includes a one-way valve that only allows aperture of fluids into the lobe. Lobe 884 is optionally filled by filling channel 892. Thereafter, the fluid may be removed from channel 892, so that the channel may be used for other tasks, while the lobe remains in its open state. Optionally, for removal of sheath assembly 880, the lobe is punctured, for example using a tool passed through channel 892. Alternatively, sheath assembly 880 is pulled out while lobe 884 is inflated.

Lobe 884 is not limited to any specific shape. Lobe 884 may be, for example, circular, rectangular or of any other shape, for example any of the shapes described in U.S. patent 6,461,294 to Oneda et al.

In some aspects of the disclosure, lobe 884 is formed of the same material as external sheath 888 optionally being produced in a same production process. Producing lobe 884 with external sheath 888 in a same production process simplifies the production, relative to a multi-stage production process.

In some aspects of the disclosure, a flexible electrode 897 is mounted on lobe 884 or on external sheath 888. Wires 896 connecting the electrode to a proximal end of sheath assembly 880 optionally run externally to external sheath 888. Alternatively or additionally, wires 896 run within channel 892 or are embedded within external sheath 888. In use, after the endoscope and sheath assembly 880 are properly positioned, lobe 884 and/or external sheath 888 are pushed radially to an open state, such that electrode 897 forms a good contact with the patient's tissue.

In some aspects of the disclosure, electrode 897 is used for sensing electrical signals. Alternatively or additionally, electrode 897 is used for ablation or otherwise applying energy to body tissue. Electrode 897 optionally comprises a suitable bio-compatible metal, such as silver.

Electrode 897 optionally includes a ring electrode which entirely surrounds lobe 884 and/or external sheath 888. Alternatively, electrode 897 has a limited radial extent. In some aspects of the disclosure, electrode 897 is located close to the distal tip of sheath assembly 880. Alternatively, electrode 897 is located away from the distal tip, for example in order not to interfere with the view of the endoscope. In some aspects of the disclosure, a plurality of electrodes are mounted on sheath assembly 880 to be pushed radially into contact by unfolding portions of the sheath assembly.

In some aspects of the disclosure, an adhesive is used to attach electrode 897 to external sheath 888. Alternatively or additionally, electrode 897 is partially embedded within external sheath 888. Electrode 897 is optionally placed externally on a first layer of external sheath 888. An additional layer of external sheath 888 covers an external frame of the electrode, leaving a window through which the electrode may contact tissue.

Sheath assembly 100 may be planned from scratch without relation to previous sheath configurations. Accordingly, both internal sheath 102 and external sheath 108 may be planned together, for example using same materials and/or same production processes. Alternatively, sheath assembly 100 is planned by adding an external sheath to a previously planned and/or tested internal sheath. For example, the internal sheath may have a design which passed barrier testing and the addition of the external sheath does not require repeated barrier testing. In accordance with this alternative, the internal and external sheath may be designed separately and/or may include different materials.

Although the above description relates to a sheath assembly for an endoscope, the sheath assembly of the present invention may be used with any other probes, including invasive probes, such as ultrasound probes, catheters and other medical devices. The sheath assemblies of the present invention may be used in substantially any body portion, including, for example, the lungs, esophagus, colon, urethra and blood vessels. The sheath assembly may be mounted on endoscopes designed for substantially any body portion, including bronchoscopes, angioscopes, cystoscopes, sigmoidoscopes, laryngoscopes, neuroscopes, mediastinoscopes and colonscopes.

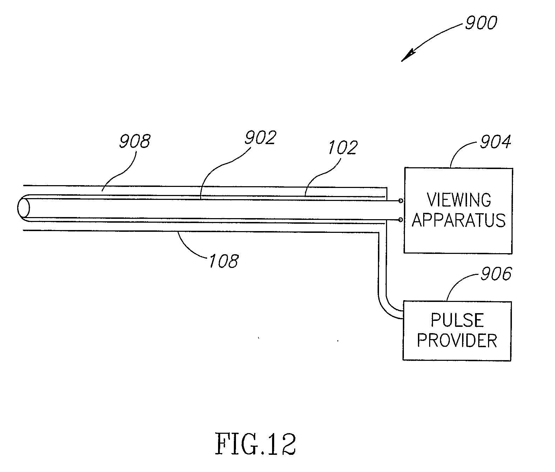

Fig. 12 is a schematic illustration of a system 900 for sensory discrimination threshold testing. System 900 includes an endoscope channel 902 and a viewing apparatus 904. A sheath assembly including an internal sheath 102 and an external sheath 108 is slid over endoscope channel 902, in order to protect the endoscope channel from contamination and to define an annular testing channel 908 between the internal and external sheaths. A pulse provider 906 is connected to testing channel 908 at its proximal end, and provides air puffs that are led through annular channel 908 to a tested site, such as the upper aero digestive tract.

In some aspects of the disclosure, the air puffs are generated by pulse provider 906 at a specific pressure and duration, according to the length, shape, materials and/or other characteristics of channel 908. Alternatively or additionally, the characteristics of sheaths 102 and 108 are selected so that the air puffs are properly provided to the test site. Pulse provider 906 is optionally constructed as described in U.S. patent 5,377,688 to Aviv.

In some aspects of the disclosure, pulse provider 906 is preprogranuned to generate different air puffs according to the characteristics of the channel through which the air puffs are provided to the test site. For example, pulse provider 906 may operate in a first state in which air puffs are generated for delivery through a circular tube and in a second state, in which air puffs are generated for delivery through an annular channel. Alternatively or additionally, different states may be determined for different channel sizes. Alternatively or additionally to preprogrammed operation states, a physician may set operation parameters of the pulse provider according to the specific leading tube used, for example based on a predetermined operation table.

It will be appreciated that the above-described methods may be varied in many ways, including, changing the order of steps, and/or performing a plurality of steps concurrently. For example, although external sheath 108 was shown as being folded in an organized, regular form, the folding may be performed without any organization, for example by crushing the material of the external sheath. It should also be appreciated that the above described description of methods and apparatus are to be interpreted as including apparatus for carrying out the methods, and methods of using the apparatus.

The present invention has been defined in the claims, and further aspects have been disclosed by way of example and are not intended to limit the scope of the invention. It should be understood that features and/or steps described with respect to one embodiment may be used with other embodiments and that not all embodiments of the invention have all of the features and/or steps shown in a particular figure or described with respect to one of the embodiments. Variations of embodiments described will occur to persons of the art. Furthermore, the terms "comprise," "include," "have" and their conjugates, shall mean, when used in the claims, "including but not necessarily limited to."

It is noted that some of the above described embodiments may describe the best mode contemplated by the inventors and therefore may include structure, acts or details of structures and acts that may not be essential to the invention and which are described as examples. Structure and acts described herein are replaceable by equivalents which perform the same function, even if the structure or acts are different, as known in the art. Therefore, the scope of the invention is limited only by the elements and limitations as used in the claims.

## Claims

1. An endoscopic sheath assembly, comprising:
an external sheath (506);
an internal sheath (502) located within the external sheath (506), a channel (516) being defined between the external sheath (506) and the internal sheath (502); and
a working tube (520) adapted to be inserted in the channel (516),
wherein the working tube (520) includes an external protrusion,
**characterized in that**:
the external sheath (506) includes a respective inner groove adapted to receive the protrusion along its length, the protrusion comprising a dovetail (522) and the groove comprising a partially closed notch (510) adapted to prevent annular escape of the protrusion from the groove; and
the external sheath (506) is formed with the inner groove comprising the partially closed notch (510).

2. An endoscopic sheath assembly according to the preceding claim, wherein the external sheath (506) circumferentially surrounds the internal sheath (502), the external sheath being foldable into a closed state in which the external sheath does not define a channel, or openable into an open state in which the external sheath defines a channel that extends along at least a portion of the elongate probe.

3. An endoscopic sheath assembly according to the preceding claim, wherein the external sheath (506) and the internal sheath (502) are connected over at least two longitudinal lines, so as to define a plurality of separate channels between the sheaths.

4. An endoscopic sheath assembly according to any of claims 2-3, wherein the external sheath (506) is heat set in the closed state.

5. An endoscopic sheath assembly according to any one of claims 2 -3, wherein the external sheath (506) is fixed in the closed state by an adhesive.

6. An endoscopic sheath assembly according to any of the preceding claims, comprising an electrode mounted on an external surface of the external sheath (506).

7. An endoscopic sheath assembly according to any of claims 2-5, wherein the external sheath (506) is folded in an unorganized manner in the closed state.

8. An endoscopic sheath assembly according to any of claims -6, wherein the external sheath (506) is pleated in the closed state.

9. An endoscopic sheath assembly according to any of claims 2-8 wherein the external sheath (506) is self-collapsible, such that it does not remain in the open state, without a force not due to the external sheath that holds it in the open state.

10. An endoscopic sheath assembly according to any of claims 2 -8, wherein the external sheath (506) does not self-collapse out of the open state, unless an external force is applied to the external sheath.

11. An endoscopic sheath assembly according to any of the preceding claims, wherein the channel does not have an aperture at its distal end.

12. An endoscopic sheath assembly according to any of the preceding claims, wherein the channel has an aperture leading out of the invasive tool, along its length.

13. An endoscopic sheath assembly according to any of the preceding claims, comprising an electrode mounted on an external surface of the external sheath.

14. An endoscopic sheath assembly according to any of the preceding claims, wherein the connection of the internal and external sheaths is non-symmetric.

15. An endoscopic sheath assembly according to any of the preceding claims, wherein the internal sheath and external sheath have a thickness of between 0.05 to 0.12 mm.

16. An endoscopic sheath assembly according to any of the preceding claims, wherein the channel is adapted for passing of fluids, tools or working tubes.

## Patentansprüche

1. Endoskopischer Hülsenaufbau mit:
einer externen Hülse (506),
einer internen Hülse (502), die innerhalb der externen Hülse (506) angeordnet ist, wobei zwischen der externen Hülse (506) und der internen Hülse (502) ein Kanal (516) definiert ist, und
einer Arbeitsröhre (520), die dazu ausgestaltet ist, um in den Kanal (516) eingesetzt zu werden,
wobei die Arbeitsröhre (520) einen externen Vorsprung aufweist,
**dadurch gekennzeichnet, dass** die externe Hülse (506) eine entsprechende innere Nut aufweist, die dazu ausgestaltet ist, um den Vorsprung entlang seiner Länge aufzunehmen, wobei der Vorsprung einen Schwalbenschwanz (522) und die Nut eine teilweise geschlossene Einkerbung (510) aufweist, die dazu ausgestaltet ist, um zu verhindern, dass der Vorsprung ringförmig aus der Nut austritt, und
dass die externe Hülse (506) mit der inneren Nut, die die teilweise geschlossene Einkerbung (510) umfasst, ausgebildet ist.

2. Endoskopische Hülsenanordnung nach einem der vorhergehenden Ansprüche, wobei die externe Hülse (506) die interne Hülse (502) in Umfangsrichtung umgibt, wobei die externe Hülse in einen geschlossenen Zustand zusammenlegbar ist, in dem die externe Hülse keinen Kanal definiert, oder in einen offenen Zustand geöffnet werden kann, in dem die externe Hülse einen Kanal definiert, der sich wenigstens entlang eines Teils der länglichen Sonde erstreckt.

3. Endoskopische Hülsenanordnung nach einem der vorhergehenden Ansprüche, wobei die externe Hülse (506) und die interne Hülse (502) über wenigstens zwei längsverlaufende Linien miteinander verbunden sind, um so eine Mehrzahl von getrennten Kanälen zwischen den Hülsen zu definieren.

4. Endoskopische Hülsenanordnung nach einem der Ansprüche 2 bis 3, wobei die externe Hülse (506) in dem geschlossenen Zustand thermofixiert ist.

5. Endoskopische Hülsenanordnung nach einem der Ansprüche 2 bis 3, wobei die externe Hülse (506) durch eine Klebung in dem geschlossenen Zustand fixiert ist.

6. Endoskopische Hülsenanordnung nach einem der vorhergehenden Ansprüche, die eine Elektrode aufweist, die an einer äußeren Oberfläche der externen Hülse (506) befestigt ist.

7. Endoskopische Hülsenanordnung nach einem der Ansprüche 2 bis 5, wobei die externe Hülse in einer unorganisierten Weise in den geschlossenen Zustand zusammenlegbar ist.

8. Endoskopische Hülsenanordnung nach einem der Ansprüche 2 bis 6, wobei die externe Hülse (506) in den geschlossenen Zustand gefaltet ist.

9. Endoskopische Hülsenanordnung nach einem der Ansprüche 2 bis 8, wobei die externe Hülse (506) sich selbst zusammenlegend ist, so dass sie ohne eine Kraft, die nicht durch die externe Hülse bedingt ist und die sie in dem offenen Zustand hält, nicht in dem offenen Zustand verbleibt.

10. Endoskopische Hülsenanordnung nach einem der Ansprüche 2 bis 8, wobei die externe Hülse (506) sich aus dem offenen Zustand nicht selbständig zusammenlegt, wenn nicht eine externe Kraft auf die externe Hülse angewendet wird.

11. Endoskopische Hülsenanordnung nach einem der vorhergehenden Ansprüche, wobei der Kanal an seinem distalen Ende keine Öffnung hat.

12. Endoskopische Hülsenanordnung nach einem der vorhergehenden Ansprüche, wobei der Kanal eine Öffnung entlang seiner Länge hat, die aus dem invasiven Werkzeug führt.

13. Endoskopische Hülsenanordnung nach einem der vorhergehenden Ansprüche, die eine Elektrode umfasst, die an einer externen Oberfläche der externen Hülse angebracht ist.

14. Endoskopische Hülsenanordnung nach einem der vorhergehenden Ansprüche, wobei die Verbindung der internen und externen Hülsen asymmetrisch ist.

15. Endoskopische Hülsenanordnung nach einem der vorhergehenden Ansprüche, wobei die interne Hülse und die externe Hülse eine Dicke zwischen 0,05 bis 0,12 mm haben.

16. Endoskopische Hülsenanordnung nach einem der vorhergehenden Ansprüche, wobei der Kanal ausgelegt ist für das Passieren von Fluida, Werkzeugen oder Arbeitsrohre.

## Revendications

1. Ensemble de gaine endoscopique, comprenant :
une gaine externe (506) ;
une gaine interne (502) située à l'intérieur de la gaine externe (506), un canal (516) étant défini entre la gaine externe (506) et la gaine interne (502) ; et
un tube de travail (520) adapté pour être inséré dans le canal (516),
dans lequel le tube de travail (520) comprend une saillie externe,
**caractérisé en ce que**
la gaine externe (506) comprend une rainure intérieure respective adaptée pour recevoir la saillie le long de sa longueur, la saillie comprenant une queue d'aronde (522), et la rainure comprenant une encoche partiellement fermée (70) adaptée pour empêcher un échappement annulaire de la saillie depuis la rainure ; et
la gaine externe (506) est formée avec la rainure intérieure comprenant l'encoche partiellement fermée (510).

2. Ensemble de gaine endoscopique selon la revendication précédente, dans lequel la gaine externe (506) entourent circonférentiellement la gaine interne (502), la gaine externe pouvant être pliée dans un état fermé dans lequel la gaine externe ne définit pas un canal, ou pouvant être ouverte dans un état ouvert dans lequel la gaine externe définit un canal qui s'étend le long d'au moins une partie de la sonde allongée.

3. Ensemble de gaine endoscopique selon la revendication précédente, dans lequel la gaine externe (506) et la gaine interne (502) sont reliées sur au moins deux lignes longitudinales, de manière à définir une pluralité de canaux séparés entre les gaines.

4. Ensemble de gaine endoscopique selon l'une quelconque des revendications 2 à 3, dans lequel la gaine externe (506) est thermofixée dans l'état fermé.

5. Ensemble formant gaine endoscopique l'une quelconque des revendications 2 à 3, dans lequel la gaine externe (506) est fixée dans l'état fermé par l'intermédiaire d'un adhésif.

6. Ensemble de gaine endoscopique selon l'une quelconque des revendications précédentes, comprenant une électrode montée sur une surface externe de la gaine externe (506).

7. Ensemble de gaine endoscopique selon l'une quelconque des revendications 2 à 5, dans lequel la gaine externe (506) est pliée d'une manière désorganisée dans l'état fermé.

8. Ensemble de gaine endoscopique selon l'une quelconque des revendications 2 à 6, dans lequel la gaine externe (506) est plissée dans l'état fermé.

9. Ensemble de gaine endoscopique selon l'une quelconque des revendications 2 à 8, dans lequel la gaine externe (506) est auto-repliable, de telle sorte qu'elle ne reste pas dans l'état ouvert sans qu'une force qui n'est pas due à la gaine externe la maintienne dans l'état ouvert.

10. Ensemble de gaine endoscopique selon l'une quelconque des revendications 2 à 8, dans lequel la gaine externe (506) ne s'auto-replie pas depuis l'état ouvert, à moins qu'une force externe ne soit appliquée à la gaine externe.

11. Ensemble de gaine endoscopique selon l'une quelconque des revendications précédentes, dans lequel le canal ne comporte pas une ouverture à son extrémité distale.

12. Ensemble de gaine endoscopique selon l'une quelconque des revendications précédentes, dans lequel le canal comporte une ouverture menant à l'extérieur de l'outil invasif, le long de sa longueur.

13. Ensemble de gaine endoscopique selon l'une quelconque des revendications précédentes, comprenant une électrode montée sur une surface externe de la gaine externe.

14. Ensemble de gaine endoscopique selon l'une quelconque des revendications précédentes, dans lequel la connexion des gaines interne et externe n'est pas symétrique.

15. Ensemble de gaine endoscopique selon l'une quelconque des revendications précédentes, dans lequel la gaine interne et la gaine externe ont une épaisseur comprise entre 0,05 et 0,12 mm.

16. Ensemble de gaine endoscopique selon l'une quelconque des revendications précédentes, dans lequel le canal est adapté pour le passage de fluides, d'outils ou de tubes de travail.
